(19) Europäisches Patentamt
European Patent Office
Office européen des brevets

(11) Veröffentlichungsnummer: **0 135 863**
**A2**

(12) # EUROPÄISCHE PATENTANMELDUNG

(21) Anmeldenummer: **84110533.1**

(22) Anmeldetag: **05.09.84**

(51) Int. Cl.[4]: **C 07 D 277/66**, C 07 D 293/12, C 07 D 215/12, C 07 D 209/12, G 03 C 1/68

(30) Priorität: **16.09.83 DE 3333450**

(43) Veröffentlichungstag der Anmeldung: **03.04.85**
**Patentblatt 85/14**

(84) Benannte Vertragsstaaten: **AT BE CH DE FR GB IT LI NL SE**

(71) Anmelder: **HOECHST AKTIENGESELLSCHAFT,**
**Postfach 80 03 20, D-6230 Frankfurt am Main 80 (DE)**

(72) Erfinder: **Dönges, Reinhard, Dr. Diplom.-Chem.,**
**Oranienstrasse 1, D-6232 Bad Soden (DE)**
Erfinder: **Ruckert, Hans, Dr. Diplom.-Chemiker,**
**Erbsenacker 21, D-6200 Wiesbaden-Naurod (DE)**
Erfinder: **Geissler, Ulrich, Dr. Diplom.-Chemiker,**
**Altenauer Strasse 34, D-6203 Hochheim/Main (DE)**
Erfinder: **Steppan, Hartmut, Dr. Diplom.-Chemiker,**
**Panoramastrasse 17, D-6200 Wiesbaden-Dotzheim (DE)**

(54) **Trihalogenmethylgruppen enthaltende Carbonylmethylenheterocyclen, Verfahren zu ihrer Herstellung und lichtempfindliches Gemisch, das diese Verbindungen enthält.**

(57) Trihalogenmethylgruppen enthaltende Carbonylmethylenheterocyclen, Verfahren zu ihrer Herstellung und lichtempfindliches Gemisch, das diese Verbindungen enthält

Es werden Verbindungen der allgemeinen Formel I

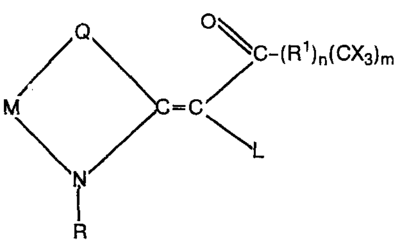

beschrieben, worin $L = H$ oder $CO-(R^1)_n(CX_3)_m$, M = Alkylen, Alkenylen oder Arylen, Q = S, Se, O, Dialkylmethylen, Alken-1,2-ylen, 1,2-Phenylen oder N-R, wobei M + Q zusammen 3 oder 4 Ringglieder bilden, R = Alkyl, Aralkyl oder Alkoxyalkyl, $R^1$ eine aromatische Gruppe, X = Cl, Br oder J bedeutet und $n = 0$ und $m = 1$ oder $n = 1$ und $m = 1$ oder 2 ist. Die Verbindungen spalten bei Belichtung HX ab und bilden Radikale. Sie sind deshalb hochwirksame Säurespender und Radikalstarter für photochemische Verfahren.



ACTORUM AG

H O E C H S T   A K T I E N G E S E L L S C H A F T
KALLE Niederlassung der Hoechst AG


5. September 1984

# Trihalogenmethylgruppen enthaltende Carbonylmethylenheterocyclen, Verfahren zu ihrer Herstellung und lichtempfindliches Gemisch, das diese Verbindungen enthält

Die Erfindung betrifft 1-Alkyl-2-carbonylmethylenheterocyclen, die an dem in 2-Stellung stehenden Substituenten mindestens eine Trichlormethylgruppe tragen, ein Verfahren zu ihrer Herstellung sowie ein lichtempfindliches Gemisch, das diese Verbindungen enthält.

Es ist bekannt, Trichlormethylgruppen enthaltende heterocyclische Verbindungen als Initiatoren für verschiedene photochemische Reaktionen einzusetzen.

Aus der DE-A 22 43 621 sind s-Triazine bekannt, die durch eine oder zwei Trichlormethylgruppen und eine chromophore Gruppe substituiert sind und als Photoinitiatoren in photopolymerisierbaren Gemischen sowie als Säurespender im Gemisch mit durch Säure spaltbaren Acetalen geeignet sind.

Ähnliche Verbindungen, in denen als chromophore Gruppe ein mindestens zweikerniger aromatischer Rest unmittelbar an den Triazinring gebunden ist, sind aus der DE-A 27 18 259 (= US-A 4 189 323) bekannt.

In der DE-A 28 51 472 sind lichtempfindliche Gemische beschrieben, die als Photoinitiatoren 2-Halogenmethyl-5-vinyl-1,3,4-oxadiazolderivate enthalten.

Aus den DE-A 30 21 590 und 30 21 599 sind mit Trichlormethylphenylgruppen substituierte Halogenoxazole bekannt, die ebenso wie alle zuvor genannten Verbindungen als Photoinitiatoren geeignet sind.

Aus der DE-B 27 17 778 sind ferner lichtempfindliche Gemische auf Basis ungesättigter Verbindungen oder polymerer Azide bekannt, die als Sensibilisatoren 2-Heteroylcarbonylmethylen-benzthiazole oder -benzselenazole enthalten.

Die bekannten Photoinitiatoren weisen die folgenden Nachteile auf:

Die Reaktionsbedingungen zur Herstellung der Verbindungen sind verhältnismäßig drastisch, so daß die Ausbeute relativ gering ist und die Bildung von unerwünschten Nebenprodukten begünstigt wird (z. B. DE-A 22 43 621, 27 18 259 oder 28 51 472); oder der Einsatz von bestimmten Katalysatoren erlaubt nur die Anwesenheit von wenigen bestimmten funktionellen Gruppen im Molekül (z. B. DE-A 27 18 259).

Bei vielen bekannten Initiatoren erfordert die unzureichende Empfindlichkeit, daß verschiedene Initiatorsysteme miteinander kombiniert werden.

Als besonders nachteilig hat es sich erwiesen, daß gerade die empfindlichsten der bekannten Initiatoren keine den Anforderungen der Praxis entsprechende Lagerfähigkeit in lichtempfindlichen Gemischen, insbesondere im Kontakt mit Kupferoberflächen aufweisen.

Aufgabe der vorliegenden Erfindung ist es, neue lichtempfindliche Verbindungen bereitzustellen, die sich in verschiedenartigen lichtempfindlichen Materialien einsetzen lassen, die einfach zugänglich sind und eine große Breite von Variationsmöglichkeiten bieten, um den Bedürfnissen auf den verschiedenen Anwendungsgebieten jeweils optimal angepaßt werden zu können; sie sollen z. B. einen breiten spektralen Empfindlichkeitsbereich aufweisen, d. h. insbesondere im nahen ultravioletten und kurzwelligen sichtbaren Bereich des Lichts empfindlich sein. Zusätzlich sollen die Verbindungen bei Einsatz in lichtempfindlichen Gemischen für die Reprographie, z. B. in Druckplatten, bereits nach der Bestrahlung einen deutlich sichtbaren Bildkontrast in der lichtempfindlichen Schicht ergeben. Ferner sollen die lichtempfindlichen Gemische, die die neuen Initiatoren enthalten, unabhängig vom Material des Schichtträgers, auf dem sie sich befinden, eine hohe Lagerstabilität aufweisen.

Die Aufgabe wird gelöst durch Verbindungen der allgemeinen Formel I

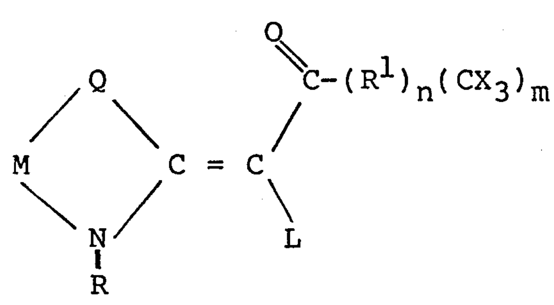

HOECHST AKTIENGESELLSCHAFT
KALLE Niederlassung der Hoechst AG



worin

L ein Wasserstoffatom oder einen Substituenten der Formel $CO-(R^1)_n(CX_3)_m$,

M einen substituierten oder unsubstituierten Alkylenrest oder Alkenylenrest oder einen 1,2-Arylenrest,

Q ein Schwefel-, Selen- oder Sauerstoffatom, eine Dialkylmethylengruppe, einen Alken-1,2-ylenrest, einen 1,2-Phenylenrest oder eine Gruppe N-R,

wobei M + Q zusammen 3 oder 4 Ringglieder bilden,

R einen Alkyl-, Aralkyl- oder Alkoxyalkylrest,

$R^1$ eine carbo- oder heterocyclische aromatische Gruppe,

X ein Chlor-, Brom- oder Jodatom bedeutet und

n = 0 und m = 1 oder

n = 1 und m = 1 oder 2 ist.

Erfindungsgemäß wird ferner ein lichtempfindliches Gemisch vorgeschlagen, das eine lichtempfindliche heterocyclische organische Verbindung (a) mit mindestens einem Trihalogenmethylsubstituenten und eine Verbindung (b) enthält, die mit dem Lichtreaktionsprodukt der organischen Verbindung (a) unter Ausbildung eines Produkts zu reagieren vermag, das eine von (b) unterschiedliche Lichtabsorption oder Löslichkeit in einem Entwickler aufweist. Das erfindungsgemäße Gemisch ist dadurch gekennzeichnet, daß die Verbindung (a) eine Verbindung der oben angegebenen Formel I ist.

H O E C H S T  A K T I E N G E S E L L S C H A F T
KALLE Niederlassung der Hoechst AG



Die erfindungsgemäßen Verbindungen bilden unter Einwirkung von aktinischer Strahlung freie Radikale, die zur Einleitung chemischer Reaktionen, insbesondere von radikalisch initiierten Polymerisationen, befähigt sind. Sie spalten ferner bei Bestrahlung Halogenwasserstoff ab, durch den säurekatalysierte Reaktionen, z. B. die Spaltung von Acetalbindungen, oder Salzbildungen, z. B. Farbumschläge von Indikatorfarbstoffen, in Gang gesetzt werden können.

In der Formel I ist vorzugsweise L ein Wasserstoffatom. M ist bevorzugt ein 1,2-Phenylenrest, der gegebenenfalls, z. B. durch Halogenatome, Carboxy-, Sulfonsäure-, Nitro-, Cyan-, Carbonyl-, Alkyl-, Aryl-, Alkoxy-, Trifluormethyl- oder Alkoxycarbonylalkylgruppen substituiert sein kann, aber bevorzugt unsubstituiert ist. M kann auch ein heterocyclischer aromatischer Rest, z. B. ein Pyridylenrest, sein. Wenn M ein mehrkerniger Arylrest ist, kann er 2 oder 3, vorzugsweise 2 Benzolkerne enthalten. M kann auch ein 1,2- oder 1,3-Alkenylenrest sein, der gegebenenfalls, z. B. durch Halogenatome, Carboxy-, Carbonyl-, Alkoxy-, Alkyl- oder Arylgruppen, substituiert ist. M kann ferner ein 1,1-, 1,2- oder 1,3-Alkylenrest sein, der auch gegebenenfalls gleichartige Substituenten tragen kann.

Q ist bevorzugt ein Schwefelatom, eine Gruppe NR oder eine Dialkylmethylengruppe mit 3 bis 13, vorzugsweise 3 bis 7, insbesondere 3 Kohlenstoffatomen. Q kann auch ein Sauerstoff- oder Selenatom, eine 1,2-Alkenylgruppe, eine

HOECHST AKTIENGESELLSCHAFT
KALLE Niederlassung der Hoechst AG



1,2-Phenylengruppe, eine Carbonyl- oder Thiocarbonylgruppe sein. Wenn Q eine Dialkylmethylengruppe ist, können die Alkylgruppen unter Ausbildung eines 5- oder 6-gliedrigen Rings miteinander verbunden sein. Wenn Q eine 1,2-Alkenylengruppe ist, kann diese u. a. mit einem oder zwei Alkyl- oder Phenylresten, Chloratomen, Alkoxygruppen oder Alkoxycarbonylgruppen substituiert sein; wenn es einen 1,2-Phenylenrest bedeutet, kann dieser als Substituenten z. B. Chloratome, Alkoxy- oder Alkoxycarbonylgruppen enthalten. Besonders bevorzugt ist Q = S, insbesondere als Bestandteil eines fünfgliedrigen Rings.

Wenn R ein Alkyl- oder Alkoxyalkylrest ist, kann dieser im allgemeinen 1 - 10, bevorzugt 1 - 6 Kohlenstoffatome enthalten. Er kann geradkettig oder verzweigt oder ggf. zu einem cycloaliphatischen Rest, z. B. einem Cyclohexylrest, ringgeschlossen sein. Beispiele für Aralkylreste sind Benzyl-, Chlorbenzyl-, Tolylmethyl- und Phenethylreste. R ist besonders bevorzugt ein Alkylrest mit 1-3 Kohlenstoffatomen.

$R^1$ ist eine ein- oder zweikernige, bevorzugt eine einkernige aromatische Gruppe, die vorzugsweise carbocyclisch ist. Beispiele für $R^1$ sind Benzol-, Naphthalin-, Triazol-, Pyrimidin-, Pyridin-, Oxazol-, Imidazol-, Thiazol-, Oxdiazol, Thiadiazol-, Furan-, Thiophen-, Pyrrol- und Isoxazolringe, die ggf. durch Halogenatome, Alkoxygruppen oder Alkylgruppen substituiert sind.

X ist bevorzugt ein Chlor- oder Bromatom, insbesondere ein Chloratom. Im allgemeinen werden Verbindungen mit n = 1 bevorzugt.

Die erfindungsgemäßen Verbindungen lassen sich vorteilhaft in Analogie zu bekannten Verfahren [z. B. A. Mistr, V. Laznicka u. M. Vavra, Coll. Czech. Chem. Commun. 36, 150 (1971)] aus einer Methylenverbindung der Formel II oder dem entsprechenden Iminiumsalz der Formel III und einem Carbonsäurehalogenid der Formel IV herstellen:

(II)

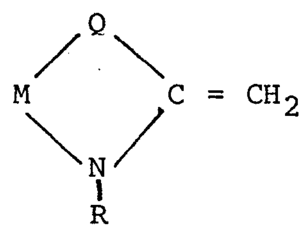

(IV)

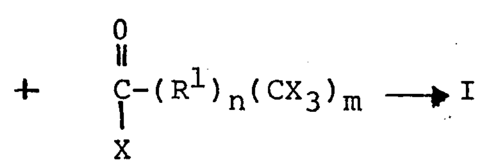

(III)

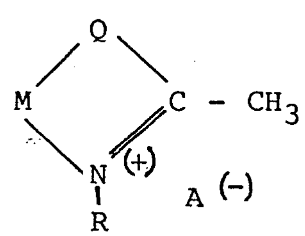

wobei A ein anorganisches Anion, bevorzugt ein Halogenidanion, Tetrafluoroboratanion, ein Perchloratanion oder ein organisches Anion, bevorzugt ein Sulfonatanion oder ein Alkylsulfatanion, bedeutet und die restlichen Symbole die oben angegebene Bedeutung haben.

Die Umsetzung findet bevorzugt unter dem Einfluß von Stickstoffbasen, z. B. Triethylamin, Dimethylbenzylamin, Diethylbenzylamin, N-Ethyldicyclohexylamin, N-Ethylpiperidin, N-Methylpiperidin, N-Methylmorpholin, N-Ethylmorpholin, N-Ethylpyrrolidon, 1,8-Diaza-bicyclo-(5,4,0)-undec-7-en, 1,4-Diaza-bicyclo-(2,2,2)-octan oder Pyridin, statt, wobei die Base selbst als Lösungsmittel dient oder ein inertes organisches Lösungsmittel zugesetzt wird, z. B. Benzol, Toluol, Dimethylformamid, Tetrahydrofuran, Diethylether, Diisopropylether oder Methylenchlorid. Die Umsetzung wird vorteilhaft bei Temperaturen zwischen 0° und 100°C durchgeführt, wobei die Menge des Carbonsäurehalogenids allgemein zwischen 1 und 4, bevorzugt zwischen 1 und 1,5 für die Herstellung von Produkten mit L = H und für die Herstellung zu Produkten mit $L = CO(R^1)_n(CX_3)_m$, zwischen 2 und 3 Mol je Mol II bzw. III liegt.

Die erfindungsgemäßen Verbindungen sind als Photoinitiatoren für photopolymerisierbare Schichten geeignet, die als wesentliche Bestandteile Monomere, Bindemittel und Initiatoren enthalten.

H O E C H S T   A K T I E N G E S E L L S C H A F T
KALLE Niederlassung der Hoechst AG



Für diese Anwendung brauchbare photopolymerisierbare Monomere sind bekannt und z. B. in den US-A 2 760 863 und 3 030 023 beschrieben.

Bevorzugte Beispiele sind Acryl- und Methacrylsäureester mehrwertiger Alkohole, wie Diglycerindiacrylat, Polyethylenglykoldimethacrylat, Acrylate und Methacrylate von Trimethylolethan, Trimethylolpropan und Pentaerythrit und von mehrwertigen alicyclischen Alkoholen. Mit Vorteil werden auch Umsetzungsprodukte von Diisocyanaten mit Partialestern mehrwertiger Alkohole eingesetzt. Derartige Monomere sind in den DE-A 20 64 079, 23 61 041 und 28 22 190 beschrieben.

Der Mengenanteil der Schicht an Monomeren beträgt im allgemeinen etwa 10 bis 80, vorzugsweise 20 bis 60 Gew.-%.

Als Bindemittel können eine Vielzahl löslicher organischer Polymerisate Einsatz finden. Als Beispiele seien genannt: Polyamide, Polyvinylester, Polyvinylacetale, Polyvinylether, Epoxidharze, Polyacrylsäureester, Polymethacrylsäureester, Polyester, Alkydharze, Polyacrylamid, Polyvinylalkohol, Polyethylenoxid, Polydimethylacrylamid, Polyvinylpyrrolidon, Polyvinylmethylformamid, Polyvinylmethylacetamid sowie Mischpolymerisate der Monomeren, die die aufgezählten Homopolymerisate bilden.

Ferner kommen als Bindemittel Naturstoffe oder umgewandelte Naturstoffe in Betracht, z. B. Gelatine und Celluloseether.

Mit Vorteil werden Bindemittel verwendet, die wasserunlöslich, aber in wäßrig-alkalischen Lösungen löslich oder mindestens quellbar sind, da sich Schichten mit solchen Bindemitteln mit den bevorzugten wäßrig-alkalischen Entwicklern entwickeln lassen. Derartige Bindemittel können z. B. die folgenden Gruppen enthalten: $-COOH$, $-PO_3H_2$, $-SO_3H$; $-SO_2NH-$, $-SO_2NHSO_2-$ und $-SO_2-NH-CO-$.

Als Beispiele hierfür seien genannt: Maleinatharze, Polymerisate aus N-(p-Tolyl-sulfonyl)-carbaminsäure-(ß-methacryloyloxy-ethyl)ester und Mischpolymerisate dieser und ähnlicher Monomerer mit anderen Monomeren sowie Styrol-Maleinsäureanhydrid-Mischpolymerisate. Alkylmethacrylat-Methacrylsäure-Mischpolymerisate und Mischpolymerisate aus Methacrylsäure, Alkylmethacrylaten und Methylmethacrylat und/oder Styrol, Acrylnitril u. a., wie sie in den DE-A 20 64 080 und 23 63 806 beschrieben sind, werden bevorzugt.

Die Menge des Bindemittels beträgt im allgemeinen 20 bis 90, vorzugsweise 40 bis 80 Gew.-% der Bestandteile der Schicht.

Die photopolymerisierbaren Gemische können je nach geplanter Anwendung und je nach den gewünschten Eigenschaften verschiedenartige Stoffe als Zusätze enthalten. Beispiele sind:

Inhibitoren zur Verhinderung der thermischen Polymerisation der Monomeren,
Wasserstoffdonatoren,
die sensitometrischen Eigenschaften derartiger Schichten modifizierende Stoffe,
Farbstoffe,
gefärbte und ungefärbte Pigmente,
Farbbildner,
Indikatoren,
Weichmacher usw.

Das photopolymerisierbare Gemisch kann für die verschiedensten Anwendungen Einsatz finden, beispielsweise zur Herstellung von Sicherheitsglas, von Lacken, die durch Licht oder Korpuskularstrahlen, z. B. Elektronenstrahlen, gehärtet werden, auf dem Dentalgebiet und insbesondere als lichtempfindliches Kopiermaterial auf dem Reproduktionsgebiet. Als Anwendungsmöglichkeiten auf diesem Gebiet seien genannt: Kopierschichten für die photomechanische Herstellung von Druckformen für den Hochdruck, den Flachdruck, den Tiefdruck, den Siebdruck, von Reliefkopien, z. B. Herstellung von Texten in Blindenschrift, von Einzelkopien, Gerbbildern, Pigmentbildern usw.. Weiter sind die Gemische zur photomechanischen Herstellung von Ätzreservagen, z. B. für die Fertigung von Namensschildern, von kopierten Schaltungen und für das Formteilätzen, anwendbar.

Die gewerbliche Verwertung des Gemischs für die genannten Anwendungszwecke kann in der Form einer flüssigen Lösung oder Dispersion, z. B. als Photoresistlösung, erfolgen, die vom Verbraucher selbst auf einen individuellen Träger, z. B. zum Formteilätzen, für die Herstellung gedruckter Schaltungen, von Siebdruckschablonen und dgl., aufgebracht wird. Das Gemisch kann auch als feste lichtempfindliche Schicht auf einem geeigneten Träger in Form eines lagerfähig vorbeschichteten lichtempfindlichen Kopiermaterials, z. B. für die Herstellung von Druckformen, vorliegen. Ebenso ist es für die Herstellung von Trockenresists geeignet.

Es ist im allgemeinen günstig, die Gemische während der Lichtpolymerisation dem Einfluß des Luftsauerstoffes weitgehend zu entziehen. Im Fall der Anwendung des Gemischs in Form dünner Kopierschichten ist es empfehlenswert, einen geeigneten, für Sauerstoff wenig durchlässigen Deckfilm aufzubringen. Dieser kann selbsttragend sein und vor der Entwicklung der Kopierschicht abgezogen werden. Für diesen Zweck sind z. B. Polyesterfilme geeignet. Der Deckfilm kann auch aus einem Material bestehen, das sich in der Entwicklerflüssigkeit löst oder mindestens an den nicht gehärteten Stellen bei der Entwicklung entfernen läßt. Hierfür geeignete Materialien sind z. B. Wachse, Polyvinylalkohol, Polyphosphate, Zucker usw..

Als Schichtträger für mit dem erfindungsgemäßen Gemisch hergestellte Kopiermaterialien sind beispielsweise Aluminium, Stahl, Zink, Kupfer und Kunststoff-Folien, z. B. aus Polyethylenterephthalat oder Cellulosacetat, sowie Siebdruckträger, wie Perlongaze, geeignet.

Die strahlungsempfindlichen Verbindungen sind als Photoinitiatoren bereits in Konzentrationen von etwa 0,1 % des Gesamtfeststoffs der Masse wirksam, eine Erhöhung über 15 % ist im allgemeinen unzweckmäßig. Vorzugsweise werden Konzentrationen von 0,2 bis 5 % verwendet.

Weiterhin können die erfindungsgemäßen Verbindungen auch in solchen strahlungsempfindlichen Gemischen eingesetzt werden, deren Eigenschaftsänderung durch saure Katalysatoren, die bei der Photolyse des Initiators entstehen, eingeleitet wird. Zu nennen sind etwa die kationische Polymerisation von Systemen, die Vinylether, N-Vinylverbindungen wie N-Vinylcarbazol oder spezielle säurelabile Lactone enthalten, wobei nicht ausgeschlossen wird, daß bei einigen dieser Reaktionen auch radikalische Vorgänge beteiligt sind. Als durch Säuren härtbare Massen sind weiterhin Aminoplaste wie Harnstoff/Formaldehydharze, Melamin/Formaldehydharze und andere N-Methylolverbindungen sowie Phenol/Formaldehydharze zu nennen. Wenn auch die Härtung von Epoxyharzen im allgemeinen durch Lewis-Säuren bzw. solche Säuren erfolgt, deren Anionen eine geringere Nukleophilie als Chlorid und Bromid besitzen, also als die Anionen der bei der Photolyse der neuen Verbindungen entstehenden Halogenwasserstoffsäuren, so härten doch Schichten, die aus Epoxyharzen und Novolaken bestehen, bei Belichtung

HOECHST AKTIENGESELLSCHAFT
KALLE Niederlassung der Hoechst AG



in Gegenwart der erfindungsgemäßen Verbindungen glatt aus.

Eine weitere vorteilhafte Eigenschaft der neuen Verbindungen besteht in ihrer Fähigkeit, in gefärbten Systemen bei der Photolyse Farbumschläge hervorzurufen; aus Farbvorläufern, z. B. Leukoverbindungen, Farbbildung zu induzieren oder bathochrome Farbverschiebungen und -vertiefungen in Gemischen zu bewirken, die Cyanin-, Merocyanin- oder Styrylfarbbasen enthalten. Auch kann z. B. in den in der DE-A 15 72 080 beschriebenen Gemischen, die Farbbase, N-Vinylcarbazol und einen Halogenkohlenwasserstoff enthalten, die Halogenverbindung Tetrabrommethan durch einen Bruchteil ihrer Menge an erfindungsgemäßer Verbindung ersetzt werden. Farbumschläge sind in der Technik auch z. B. bei der Herstellung von Druckformen erwünscht, um nach der Belichtung bereits vor der Entwicklung das Kopierergebnis beurteilen zu können.

Statt der in den DE-A 23 31 377 und 26 41 100 genannten Säurespender sind vorteilhaft die vorliegenden Verbindungen zu benutzen.

Ein besonders bevorzugtes Anwendungsgebiet für die erfindungsgemäßen Verbindungen sind Gemische, die neben ihnen als wesentliche Komponente eine Verbindung mit mindestens einer durch Säure spaltbaren C-O-C-Gruppierung enthalten. Als durch Säure spaltbare Verbindungen sind in erster Linie zu nennen:

A) solche mit mindestens einer Orthocarbonsäureester- und bzw. oder Carbonsäureamidacetalgruppierung, wobei die Verbindungen auch polymeren Charakter haben und die genannten Gruppierungen als verknüpfende Elemente in der Hauptkette oder als seitenständige Substituenten auftreten können und

B) Polymerverbindungen mit wiederkehrenden Acetal- und/ oder Ketalgruppierungen.

Durch Säure spaltbare Verbindungen des Typs A als Komponenten strahlungsempfindlicher Gemische sind in den DE-A 26 10 842 oder 29 28 636 ausführlich beschrieben; Gemische, die Verbindungen des Typs B enthalten, sind Gegenstand der DE-C 27 18 254.

Als durch Säure spaltbare Verbindungen sind z. B. auch die speziellen Aryl-alkyl-acetale und -aminale der DE-C 23 06 248 zu nennen, die durch die Photolyseprodukte der erfindungsgemäßen Verbindungen ebenfalls abgebaut werden.

Solche Gemische, in denen durch Einwirkung von aktinischer Strahlung mittelbar oder unmittelbar Moleküle in kleinere umgewandelt werden, weisen an den bestrahlten Stellen im allgemeinen eine erhöhte Löslichkeit, Klebrigkeit oder Flüchtigkeit auf. Diese Partien können durch geeignete Maßnahmen, beispielsweise Herauslösen mit einer Entwicklungsflüssigkeit, entfernt werden. Bei

Kopiermaterialien spricht man in diesen Fällen von positiv arbeitenden Systemen.

Die bei vielen Positiv-Kopiermaterialien bewährten Novolak-Kondensationsharze haben sich als Zusatz auch bei der Anwendung der erfindungsgemäßen Verbindungen in Gemischen mit durch Säure spaltbaren Verbindungen als besonders brauchbar und vorteilhaft erwiesen. Sie fördern die starke Differenzierung zwischen den belichteten und unbelichteten Schichtpartien beim Entwickeln, besonders die höher kondensierten Harze mit substituierten Phenolen als Formaldehyd-Kondensationspartner. Die Art und Menge der Novolak-Harze kann je nach Anwendungszweck verschieden sein; bevorzugt sind Novolak-Anteile am Gesamtfeststoff zwischen 30 und 90, besonders bevorzugt 55 - 85 Gew.-%.

Zusätzlich können noch zahlreiche andere Harze mitverwendet werden, bevorzugt Vinylpolymerisate wie Polyvinylacetate, Polyacrylate, Polyvinylether und Polyvinylpyrrolidone, die selbst durch Comonomere modifiziert sein können. Der günstigste Anteil an diesen Harzen richtet sich nach den anwendungstechnischen Erfordernissen und dem Einfluß auf die Entwicklungsbedingungen und beträgt im allgemeinen nicht mehr als 20 % vom Novolak. In geringen Mengen kann das lichtempfindliche Gemisch für spezielle Erfordernisse wie Flexibilität, Haftung und Glanz etc. außerdem noch Substanzen wie Polyglykole, Cellulose-Derivate wie Ethylcellulose, Netzmittel, Farbstoffe und feinteilige Pigmente sowie bei Bedarf

UV-Absorber enthalten. Entwickelt wird vorzugsweise mit in der Technik üblichen wäßrig-alkalischenn Entwicklern, die auch kleine Anteile organischer Lösemittel enthalten können, oder aber mit organischen Lösemitteln.

Die bereits im Zusammenhang mit den photopolymerisierbaren Gemischen aufgeführten Träger kommen ebenfalls für positiv arbeitende Kopiermaterialien in Frage, zusätzlich die in der Mikroelektronik üblichen Silizium- und Siliziumdioxidoberflächen.

Die Menge der als Photoinitiator eingesetzten erfindungsgemäßen Verbindungen kann in den positiv arbeitenden Gemischen je nach Substanz und Schicht sehr verschieden sein. Günstigere Ergebnisse werden erhalten zwischen etwa 0,1 und 10 %, bezogen auf den Gesamtfeststoff, bevorzugt sind etwa 0,2 bis 5 %. Für Schichten mit Dicken über 10 µm empfiehlt es sich, relativ wenig Säurespender zu verwenden.

Grundsätzlich ist elektromagnetische Strahlung mit Wellenlängen bis etwa 600 nm zur Belichtung geeignet, Der bevorzugte Wellenlängenbereich erstreckt sich von 250 bis 500 nm.

Die Vielfalt der erfindungsgemäßen Verbindungen, deren Absorptionsmaxima teilweise noch weit im sichtbaren Teil des Spektrums zu finden sind und deren Absorptionsbereich über 500 nm hinausreichen kann, gestattet es, den Photoinitiator optimal auf die verwendete Lichtquelle abzustimmen. Als Lichtquellen sind beispielsweise zu nennen:

Röhrenlampen, Xenonimpulslampen, metallhalogeniddotierte Quecksilberdampf-Hochdrucklampen und Kohlebogenlampen.

Darüber hinaus ist bei den erfindungsgemäßen lichtempfindlichen Gemischen das Belichten in üblichen Projektions- und Vergrößerungs-Geräten unter dem Licht der Metallfadenlampen und Kontakt-Belichtung mit gewöhnlichen Glühbirnen möglich. Die Belichtung kann auch mit kohärentem Licht eines Lasers erfolgen. Geeignet für die Zwecke vorliegender Erfindung sind leistungsgerechte kurzwellige Laser, beispielsweise Argon-Laser, Krypton-Ionen-Laser, Farbstoff-Laser und Helium-Cadmium-Laser, die insbesondere zwischen 250 und 500 nm emittieren. Der Laserstrahl wird mittels einer vorgegebenen programmierten Strich- und/oder Raster-Bewegung gesteuert.

Das Bestrahlen mit Elektronenstrahlen ist eine weitere Differenzierungsmöglichkeit. Elektronenstrahlen können Gemische, die eine der erfindungsgemäßen Verbindungen und eine durch Säure spaltbare Verbindung enthalten, wie auch viele andere organische Materialien durchgreifend zersetzen und vernetzen, so daß ein negatives Bild entsteht, wenn die unbestrahlten Teile durch Lösemittel oder Belichten ohne Vorlage und Entwickeln entfernt werden.

Bei geringerer Intensität und/oder höherer Schreibgeschwindigkeit des Elektronenstrahls bewirkt dagegen der Elektronenstrahl eine Differenzierung in Richtung höherer Löslichkeit, d. h. die bestrahlten Schichtpartien können

vom Entwickler entfernt werden. Die günstigsten Bedingungen können durch Vorversuche leicht ermittelt werden.

Bevorzugte Anwendung finden die eine der erfindungsgemäßen Verbindungen enthaltenden strahlungsempfindlichen Gemische bei der Herstellung von Druckformen, d. h. insbesondere Offset-, autotypischen Tiefdruck- und Siebdruckformen, in Photoresistlösungen und in sogenannten Trockenresists.

Die folgenden Beispiele dienen zur näheren Erläuterung der Erfindung, es erfolgt zunächst die Beschreibung der Herstellung von verschiedenen erfindungsgemäßen Verbindungen, woran sich die Anwendung von einigen dieser Verbindungen in strahlungsempfindlichen Gemischen anschließt.

In den Beispielen stehen Gew.-Teile (Gt) und Vol.-Teile (Vt) im Verhältnis von g zu ml. Prozent- und Mengenangaben sind, wenn nichts anderes angegeben ist, in Gewichtseinheiten zu verstehen.

HOECHST AKTIENGESELLSCHAFT
KALLE Niederlassung der Hoechst AG



Tabelle I

Verbindungen der Formel I mit
Q = S; $R^1$ = Benzolring

| Verb. Nr. | R | M | L | n | $(CX_3)_m$ |
|---|---|---|---|---|---|
| 1 | $C_2H_5$ | 1,2-Phenylen | H | 1 | $4\text{-}CCl_3$ |
| 1a | $C_2H_5$ | $5\text{-}CH_3$-1,2-Phenylen | H | 1 | $4\text{-}CCl_3$ |
| 1b | $CH_2C_6H_5$ | 1,2-Phenylen | H | 1 | $4\text{-}CCl_3$ |
| 2 | $C_2H_5$ | " | RX | 1 | $4\text{-}CCl_3$ |
| 3 | $C_2H_5$ | " | H | 1 | $3\text{-}CCl_3$ |
| 4 | $CH_3$ | " | H | 1 | $4\text{-}CCl_3$ |
| 5(V) | $C_2H_5$ | " | H | 1 | $4\text{-}CF_3$ |
| 6 | $C_2H_5$ | " | H | 0 | $CCl_3$ |
| 6a | $CH_3$ | " | H | 0 | $CCl_3$ |
| 6b | $C_2H_5$ | $5\text{-}CH_3$-1,2-Phenylen | H | 0 | $CCl_3$ |
| 6c | $CH_2C_6H_5$ | 1,2-Phenylen | H | 0 | $CCl_3$ |
| 7 | $C_2H_5$ | " | H | 1 | $3,5\text{-}(CCl_3)_2$ |
| 8 | $C_2H_4OCH_3$ | " | H | 1 | $4\text{-}CCl_3$ |
| 8a | $(CH_2)_5CH_3$ | " | H | 1 | $4\text{-}CCl_3$ |
| 9(V) | $C_2H_5$ | " | H | 1 | --- |

(V) = Vergleichsverbindung
Ph = Phenylen
RX = $CO\text{-}Ph\text{-}CCl_3(p)$

HOECHST AKTIENGESELLSCHAFT
KALLE Niederlassung der Hoechst AG



## Tabelle II

Verbindungen der Formel I mit
Q = S; M = 1,2-Naphthylen; R = $CH_3$

| Verb. Nr. | $R^1$ | L | n | $(CX_3)_m$ |
|---|---|---|---|---|
| 10 | 1,4-Phenylen | H | 1 | $CCl_3$ |
| 10a | 1,3,5-Phentriyl | H | 1 | $(CCl_3)_2$ |
| 11(V) | 1,4-Phenylen | H | 1 | $CF_3$ |
| 12(V) | Furyl-(2) | Furyl-(2)-carbonyl | 1 | --- |
| 13 | --- | H | 0 | $CCl_3$ |
| 14(V) | Phenyl | H | 1 | --- |

## Tabelle III

Verbindungen der Formel I mit
Q = $C(CH_3)_2$; L = H; X = Cl; R = $CH_3$

| Verb. Nr. | M | $R^1$ | n | m |
|---|---|---|---|---|
| 15 | 1,2-Phenylen | 1,4-Phenylen | 1 | 1 |
| 15a | 5-Cl-1,2-Phenylen | " | 1 | 1 |
| 15b | 1,2-Phenylen | 1,3-Phenylen | 1 | 1 |
| 16 | 1,2-Phenylen | --- | 0 | 1 |
| 16a | 5-Cl-1,2-Phenylen | --- | 0 | 1 |
| 17 | 1,2-Phenylen | 1,3,5-Phentriyl | 1 | 2 |

HOECHST AKTIENGESELLSCHAFT
KALLE Niederlassung der Hoechst AG



Tabelle IV

Verbindungen der Formel I mit
Q = S; L = H; X = Cl; $R^1$ = 1,4-Phenylen; R = $C_2H_5$

| Verb. Nr. | M | n |
|---|---|---|
| 18 | $C_2H_5OCO-C-$<br>‖<br>$CH_3-C-$ | 1 |
| 19 | $C_2H_5OCO-C-$<br>‖<br>$CH_3-C-$ | 0 |
| 20 | Phenyl-C-<br>‖<br>Phenyl-C- | 1 |
| 20a | Phenyl-C-<br>‖<br>H-C- | 1 |
| 21 | Phenyl-C-<br>‖<br>Phenyl-C- | 0 |
| 21a | Phenyl-C-<br>‖<br>H-C- | 0 |

HOECHST AKTIENGESELLSCHAFT
KALLE Niederlassung der Hoechst AG



Tabelle V

Verbindungen der Formel I mit
Q = Ethen-1,2-ylen; M = 1,2-Phenylen; R = $CH_3$; n = 0;
X = Cl

| Verb. Nr. | L |
|---|---|
| 22 | H |
| 23 | Trichloracetyl |

Tabelle VI

Verbindungen der Formel I mit
Q = Se; L = H; X = Cl; $R^1$ = 1,4-Phenylen; R = $C_2H_5$; m = 1

| Verb. Nr. | M | n |
|---|---|---|
| 24 | 1,2-Phenylen | 1 |
| 25 | 5-$CH_3$-1,2-Phenylen | 1 |
| 26 | 5-$CH_3O$-1,2-Phenylen | 1 |
| 27 | 1,2-Phenylen | 0 |

Herstellungsbeispiel 1
Herstellung der Verbindung 1

A) 3-Ethyl-2-methyl-benzthiazolium-p-toluolsulfonat

Unter Rühren werden 300 g (2 mol) 2-Methylbenzthiazol und 440 g (2,2 mol) p-Toluolsulfonsäure-ethylester (oder ein Gemisch aus o- und p-Toluolsulfonsäure-ethylester) auf

HOECHST AKTIENGESELLSCHAFT
KALLE Niederlassung der Hoechst AG



150°C erhitzt, wobei durch exotherme Reaktion die Temperatur auf ca. 200°C steigt. Nach 10 Minuten wird in 2 Liter Aceton gegossen, das ausgefallene Produkt abgesaugt, mit Aceton gewaschen und im Vakuum getrocknet. Ausbeute 730 g (98 %).

B) 2-(p-Trichlormethyl-benzoylmethylen)-3-ethyl-benzthiazolin (Verbindung 1)

35 g (0,1 mol) 3-Ethyl-2-methyl-benzthiazolium-p-toluolsulfonat werden in 300 ml Toluol suspendiert, 28 g (0,11 mol) p-Trichlormethyl-benzoylchlorid zugegeben und bei 15 Grad 23 g (0,23 mol; 31,5 ml) Triethylamin zugetropft.

Nach 3 - 4 Stunden bei Raumtemperatur wird das ausgefallene Produkt abgesaugt und mit etwas Methanol gewaschen.

Es wird aus Ethanol, Essigester, Aceton oder Acetonitril umkristallisiert.
Ausbeute: 34 g (85 %).
Schmp.: 174-176°C
$C_{18}H_{14}Cl_3NOS$: Ber: C 54,22; H 3,54; N 3,51; Cl 26,67
MG: 398,74 Gef: C 54,2 ; H 3,4 ; N 3,5 ; Cl 26,4
UV in Dimethylformamid (DMF): 396 nm (30900)

H O E C H S T A K T I E N G E S E L L S C H A F T
KALLE Niederlassung der Hoechst AG



Entsprechend werden hergestellt:

Verbindung 1a: 2-(p-Trichlormethyl-benzoylmethylen)-3-ethyl-5-methyl-benzthiazolin
Schmp.: 181-183°C

$C_{19}H_{16}Cl_3NOS$: Ber: C 55,29; H 3,91; N 3,39; Cl 25,77
MG: 412,77 Gef: C 55,0 ; H 4,0 ; N 3,3 ; Cl 25,6
UV (in DMF): 398 nm (32800)

Verbindung 1b: 2-(p-Trichlormethyl-benzoylmethylen)-3-benzyl-benzthiazolin
Schmp.: 182-185°C

$C_{23}H_{16}Cl_3NOS$: Ber: C 59,95; H 3,50; N 3,04; Cl 23,68
MG: 460,81 Gef: C 59,8 ; H 3,5 ; N 3,0 ; Cl 23,0
UV (in DMF): 395 nm (31500)

Verbindung 3: 2-(m-Trichlormethyl-benzoylmethylen)-3-ethyl-benzthiazolin
Schmp.: 157 - 159°C

$C_{18}H_{14}Cl_3NOS$: Ber: C 54,22; H 3,54; N 3,51; Cl 26,67
MG: 398,74 Gef: C 54,3 ; H 3,6 ; N 3,5 ; Cl 26,4
UV (in DMF): 388 nm (46900)

Verbindung 4: 2-(p-Trichlormethyl-benzoylmethylen)-3-methyl-benzthiazolin
(aus 2,3-Dimethylbenzthiazolium-p-toluolsulfonat)

Schmp.: 194-196°C
$C_{17}H_{12}Cl_3NOS$: Ber: C 53,08; H 3,14; N 3,64; Cl 27,65
MG: 384,71 Gef: C 54,0 ; H 3,4; N 3,6 ; Cl 27,2
UV(in DMF): 395 nm (32200)

Verbindung 5: 2-(p-Trifluormethyl-benzoylmethylen)-3-ethyl-benzthiazolin
(Vergleichsverbindung)
Schmp.: 179-180°C
$C_{18}H_{14}F_3NOS$ Ber: C 61,88; H 4,04; N 4,01
MG: 349,37 Gef: C 62,1 ; H 4,1 ; N 4,1
UV(in DMF): 392 nm (31300)

Verbindung 6a:
2-(Trichloracetylmethylen)-3-methyl-benzthiazolin
Schmp.: 189-190°C
$C_{11}H_8Cl_3NOS$ Ber: C 42,81; H 2,61; N 4,45; Cl 34,46
MG: 308,62 Gef: C 42,5 ; H 2,5 ; N 4,3 ; Cl 34,5
UV (in DMF): 369 nm (28700)

Verbindung 7: 2-[3,5-Bis-(trichlormethyl)-benzoylmethylen]-3-ethyl-benzthiazolin
Schmp.: 180-183°C

$C_{19}H_{13}Cl_6NOS$: Ber: C 44,22; H 2,54; N 2,71; Cl 41,22
MG: 516,10 Gef: C 44,2 ; H 2,4 ; N 2,7 ; Cl 40,6
UV(in DMF): 399 nm (30 700)

Verbindung 8: 2-(p-Trichlormethyl-benzoylmethylen)-3-(2-methoxyethyl)-benzthiazolin
(aus 2-Methyl-3-(2-methoxyethyl)-benzthiazolium-p-toluolsulfonat)
Schmp.: 162-165°C

$C_{19}H_{16}Cl_3NO_2S$: Ber: C 53,22; H 3,76; N 3,27; Cl 24,81
MG: 428,77 Gef: C 53,4 ; H 3,9 ; N 3,2 ; Cl 24,9
UV(in DMF): 396 nm (31 000)

Verbindung 8a: 2-(p-Trichlormethyl-benzoylmethylen)-3-n-hexyl-benzthiazolin
Schmp.: 111-113°C

$C_{22}H_{22}Cl_3NOS$: Ber: C 58,10; H 4,88; N 3,08; Cl 23,38
MG: 454,85 Gef: C 58,4 ; H 4,9 ; N 3,0 ; Cl 23,1
UV (in DMF): 397 nm (32100)

Verbindung 9: 2-Benzoylmethylen-3-ethyl-benzthiazolin
(Vergleichsverbindung)
Schmp.: 139-140°C

$C_{17}H_{15}NOS$: Ber: C 72,57; H 5,37; N 4,98
MG: 281,38 Gef: C 72,6 ; H 5,6 ; N 5,0
UV(in DMF) 381 nm (36 600)

Verbindung 10: 2-(p-Trichlormethyl-benzoylmethylen)-3-methyl-naphtho[1,2-d]thiazolin
(aus 2,3-Dimethyl-naphtho[1,2-d]thiazolium-p-toluolsulfonat)

HOECHST AKTIENGESELLSCHAFT
KALLE Niederlassung der Hoechst AG



Schmp.: 239-242°C

$C_{21}H_{14}Cl_3NOS$: Ber: C 58,01; H 3,25; N 3,22; Cl 24,46
MG: 434,77 Gef: C 57,7 ; H 3,3 ; N 2,9 ; Cl 24,3
UV (in DMF): 412 nm (35 200)

Verbindung 10a: 2-(3,5-Bis-trichlormethyl-benzoylmethylen)-3-methyl-naphtho[1,2-d]thiazolin
Schmp.: 241-242°C

$C_{22}H_{13}Cl_6NOS$: Ber: C 47,86; H 2,37; N 2,54; Cl 38,53
MG: 552,14 Gef: C 47,6 ; H 2,3 ; N 2,2 ; Cl 38,5
UV (in DMF): 415 nm (33 400)

Verbindung 11: (Vergleichsverbindung) 2-(p-Trifluormethyl-benzoylmethylen)-3-methyl-naphtho[1,2-d]thiazolin (aus 2,3-Dimethyl-naphtho[1,2-d]thiazolium-p-toluolsulfonat)
Schmp.: 252-254°C

$C_{21}H_{14}F_3NOS$: Ber: C 65,45; H 3,66; N 3,63
MG: 385,41 Gef: C 64,7 ; H 3,8 ; N 3,6
UV(in DMF): 409 nm (34 200)

Verbindung 13: 2-Trichloracetylmethylen-3-methyl-naphtho-[1,2-d]thiazolin
(aus 2,3-Dimethyl-naphtho[1,2-d]thiazolium-p-toluolsulfonat)
Schmp.: 263°C

$C_{15}H_{10}Cl_3NOS$: Ber: C 50,23; H 2,81; N 3,91; Cl 29,65
MG: 358,67 Gef: C 50,2 ; H 2,9 ; N 3,9 ; Cl 30,2
UV(in DMF): 387 nm (35 100)

Verbindung 14: 2-Benzoylmethylen-3-methyl-naphtho[1,2-d]-thiazolin
(Vergleichsverbindung) Schmp.: 220-222°C

$C_{20}H_{15}NOS$: Ber: C 75,68; H 4,76; N 4,41
MG: 317,41 Gef: C 75,4 ; H 4,8 ; N 3,9
UV(in DMF): 331 nm (4900), 399 nm (38900)

Verbindung 24: 2-(p-Trichlormethyl-benzoylmethylen)-3-ethyl-benzoselenazolin
Schmp.: 175-178°C

$C_{18}H_{14}Cl_3NOSe$: Ber: C 48,51; H 3,17 ; N 3,14 ; Cl 23,87
MG: 445,77 Gef: C 48,3 ; H 3,2 ; N 3,1 ; Cl 23,6
UV (in DMF): 399 nm (29900)

Verbindung 25: 2-(p-Trichlormethyl-benzoylmethylen)-3-ethyl-5-methyl-benzoselenazolin
Schmp.: 195-197°C

$C_{19}H_{16}Cl_3NOSe$: Ber: C 49,65; H 3,51; N 3,05; Cl 23,14
MG: 459,66 Gef: C 49,9 ; H 3,7 ; N 3,1 ; Cl 23,0
UV (in DMF): 401 nm (30800)

Verbindung 26: 2-(p-Trichlormethyl-benzoylmethylen)-3-ethyl-5-methoxy-benzoselenazolin
Schmp.: 174-176°C
$C_{19}H_{16}Cl_3NO_2Se$: Ber: C 47,98; H 3,39; N 2,94; Cl 22,36
MG: 475,66 Gef: C 48,0 ; H 3,4 ; N 2,9 ; Cl 22,1
UV (in DMF): 407 nm (30100)

Verbindung 27: 2-Trichloracetylmethylen-3-ethyl-benzoselenazolin
Schmp.: 139-140°C
$C_{12}H_{10}Cl_3NOSe$: Ber: C 39,00; H 2,73; N 3,79; Cl 28,78
MG: 369,54 Gef: C 39,0 ; H 2,8 ; N 3,8 ; Cl 28,5
UV (in DMF): 371 nm (29500)

Herstellungsbeispiel 2

Herstellung der Verbindung 15:
2-(p-Trichlormethyl-benzoylmethylen)-1,3,3-trimethyl-indolin

Zu 13,48 g (80 mmol) 1,3,3-Trimethyl-2-methylen-indolin (Tribase) und 26 ml (188 mmol) Triethylamin, gelöst in 300 ml wasserfreiem Toluol, werden bei 0-5°C 10,3 ml (16,6 g; 92 mmol) Trichloracetylchlorid getropft. Es wird 3 Stunden bei Raumtemperatur nachgerührt, vom Niederschlag abfiltriert, das Filtrat mit Wasser gewaschen, über Natriumsulfat getrocknet und eingeengt. Das Produkt wird zweimal aus Diisopropylether umkristallisiert.
Ausbeute 12,6 g (49 %)
Schmp.: 170-171°C

$C_{20}H_{18}Cl_3NO$: Ber: C 60,86; H 4,60; N 3,55; Cl 26,94
MG: 394,72 Gef: C 61,0 ; H 4,6 ; N 3,6 ; Cl 26,6
UV(in DMF): 395 nm (26300)

Analog werden hergestellt:

Verbindung 15a: 2-(p-Trichlormethyl-benzoylmethylen)-1,3,3-trimethyl-5-chlor-indolin
Schmp.: 164-171°C

$C_{20}H_{17}Cl_4NO$: Ber: C 55,97; H 3,99; N 3,26; Cl 33,04
MG: 429,18 Gef: C 55,6 ; H 3,9 ; N 3,2 ; Cl 33,2
UV (in DMF): 394 nm (28700)

Verbindung 15b: 2-(m-Trichlormethyl-benzoylmethylen)-1,3,3-trimethyl-indolin
Schmp.: 161-165°C

$C_{20}H_{18}Cl_3NO$: Ber: C 60,86; H 4,60; N 3,55; Cl 26,94
MG: 394,72 Gef: C 61,1 ; H 4,6 ; N 3,6 ; Cl 26,8
UV (in DMF): 388 nm (28100)

Verbindung 16: 2-Trichloracetylmethylen-1,3,3-trimethyl-indolin
Schmp.: 101°C

$C_{14}H_{14}Cl_3NO$: Ber: C 52,77; H 4,43; N 4,40; Cl 33,38
MG: 318,63 Gef: C 53,1 ; H 4,4 ; N 4,4 ; Cl 33,1

UV(in DMF): 375 nm (25900)

Verbindung 16a: 2-Trichloracetylmethylen-1,3,3-trimethyl-5-chlor-indolin
Schmp.: 163-165°C

$C_{14}H_{13}Cl_4NO$: Ber: C 47,63; H 3,71; N 3,97; Cl 40,16
MG: 353,08 Gef: C 47,9 ; H 3,7 ; N 4,0 ; Cl 40,2
UV (in DMF): 376 nm (28 000)

Verbindung 17: 2-[3,5-Bis-(trichlormethyl)-benzoylmethylen]-1,3,3-trimethyl-indolin
(aus 2-Methylen-1,3,3-trimethyl-indolin)
Schmp.: 185-186°C

$C_{21}H_{17}Cl_6NO$: Ber: C 49,26; H 3,35; N 2,74; Cl 41,54
MG: 512,09 Gef: C 49,6 ; H 3,6 ; N 2,6 ; Cl 40,9
UV (in DMF): 398 nm (24 600)

Herstellungsbeispiel 3

Herstellung der Verbindung 2:
2-[Bis-(p-trichlormethylbenzoyl)-methylen]-3-ethyl-benzthiazolin

Zu 7 g (20 mmol) 2-Methyl-3-ethylbenzthiazolium-p-toluolsulfonat in 30 ml trockenem Pyridin werden bei 5 - 15 °C 12 g (46,5 mmol) p-Trichlormethyl-benzoylchlorid getropft. Es wird 2 Stunden auf 100°C erhitzt. Nach dem Abkühlen wird das Pyridin im Vakuum abdestilliert und der Rückstand mit 80 ml Methanol versetzt. Das ausgefallene Produkt wird aus Acetonitril umkristallisiert.
Ausbeute: 7,5 g (60 %)
Schmp.: 205-207°C

HOECHST AKTIENGESELLSCHAFT
KALLE Niederlassung der Hoechst AG



$C_{26}H_{17}Cl_6NO_2S$: Ber: C 50,35; H 2,76; N 2,26; Cl 34,30
MG: 620,21 Gef: C 50,6 ; H 3,0 ; N 2,3 ; Cl 34,4
UV(in DMF): 372 nm (25300)

Entsprechend wird hergestellt:

Verbindung 12: 2-[Bis-(2-furoyl)-methylen]-3-methyl-naphtho[1,2-d]thiazolin
(Vergleichsverbindung) (aus 2,3-Dimethyl-naphtho[1,2-d]thiazolium-p-toluolsulfonat)
Schmp.: 205-206°C

$C_{23}H_{15}NO_4S$ Ber: C 68,82; H 3,77; N 3,49
MG: 401,44 Gef: C 68,7 ; H 3,9 ; N 3,5
UV(in DMF): 408 nm (35 000)

Herstellungsbeispiel 4
Herstellung der Verbindung 6:
2-Trichloracetylmethylen-3-ethyl-benzthiazolin

10 g (28,6 mmol) 2-Methyl-3-ethyl-benzthiazolium-p-toluolsulfonat werden in 150 ml Toluol suspendiert, dann werden 6,8 g (67,2 mmol) Triethylamin zugegeben und bei 5 - 10°C 6 g (33 mmol) Trichloracetylchlorid, gelöst in etwas Toluol, zugetropft. Nach 3 Stunden bei Raumtemperatur wird von den Ammoniumsalzen abgesaugt, die Reaktionslösung mit Wasser gewaschen, über Natriumsulfat getrocknet und im Vakuum eingeengt. Das Produkt wird aus Diisopropylether zur Kristallisation gebracht.

Ausbeute: 7,2 g (78 %)
Schmp.: 136-139°C

$C_{12}H_{10}Cl_3NOS$: Ber: C 44,67; H 3,12; N 4,34; Cl 32,97
MG: 322,64 Gef: C 44,8 ; H 3,2 ; N 4,2 ; Cl 33,1
UV(in DMF): 369 nm (31 600)

Entsprechend werden hergestellt:

Verbindung 6b: 2-Trichloracetylmethylen-3-ethyl-5-methyl-benzthiazolin
Schmp.: 199-200°C

$C_{13}H_{12}Cl_3NOS$: Ber: C 46,38; H 3,59; N 4,16; Cl 31,59
MG: 336,67 Gef: C 46,4 ; H 3,7 ; N 4,0 ; Cl 31,3
UV (in DMF): 370 nm (28500)

Verbindung 6c: 2-Trichloracetylmethylen-3-benzyl-benzthiazolin
Schmp.: 197-198°C

$C_{17}H_{12}Cl_3NOS$: Ber: C 53,08; H 3,14; N 3,64; Cl 27,65
MG: 384,71 Gef: C 53,2 ; H 3,2 ; N 3,6 ; Cl 27,5
UV (in DMF): 369 nm (32400)

Herstellungsbeispiel 5

Herstellung der Verbindung 18:

2-(p-Trichlormethyl-benzoylmethylen)-3-ethyl-4-methyl-5-ethoxycarbonyl-thiazolin

A) 2,4-Dimethyl-3-ethyl-5-ethoxycarbonyl-thiazolium-p-toluolsulfonat
2-Chlor-acetessigsäureethylester wird mit Thioacetamid zu 2,4-Dimethyl-5-ethoxycarbonyl-thiazolin kondensiert, das analog Herstellungsbeispiel 1 A mit p-Toluolsulfonsäureethylester zum p-Toluolsulfonat der quaternären Ammoniumbase umgesetzt wird.

B) Verbindung 18:

Analog Herstellungsbeispiel 1 B wird das 2,4-Dimethyl-3-ethyl-5-ethoxycarbonyl-thiazolium-p-toluolsulfonat mit p-Trichlormethyl-benzoylchlorid zum 2-(p-Trichlormethyl-benzoylmethylen)-3-ethyl-4-methyl-5-ethoxycarbonyl-thiazolin umgesetzt.

Schmp.: 214-216°C

| | | | | | |
|---|---|---|---|---|---|
| $C_{18}H_{18}Cl_3NO_3S$ | Ber.: | C 49,73; | H 4,17; | N 3,22; | Cl 24,46 |
| MG: 434,77 | Gef.: | C 49,7 ; | H 4,2 ; | N 3,2 ; | Cl 24,1 |

UV (im DMF): 317 nm (5300), 402 nm (29 300)

Analog werden hergestellt:

Verbindung 19:

2-Trichloracetylmethylen-3-ethyl-4-methyl-5-ethoxycarbonyl-thiazolin
Schmp.: 144-146°C

$C_{12}H_{14}Cl_3NO_3S$ Ber.: C 40,19; H 3,93; N 3,91; Cl 29,65
MG: 358,67 Gef.: C 40,0 ; H 3,9 ; N 3,7 ; Cl 29,6
UV (in DMF): 295 nm (6100), 376 nm (26 700)

Verbindung 20:

2-(p-Trichlormethyl-benzoylmethylen)-3-ethyl-4,5-diphenyl-thiazolin
Schmp.: 210-211°C

$C_{26}H_{20}Cl_3NOS$ Ber.: C 62,35; H 4,02; N 2,80; Cl 21,23
MG: 500,88 Gef.: C 62,2 ; H 4,2 ; N 2,6 ; Cl 21,1
UV (in DMF): 322 nm (5900); 412 nm (27400)

Verbindung 20a: 2-(p-Trichlormethyl-benzoylmethylen)-3-ethyl-5-phenyl-thiazolin
Schmp.: 172-175°C

$C_{20}H_{16}Cl_3NOS$: Ber: C 56,55; H 3,80; N 3,30; Cl 25,04
MG: 424,78 Gef: C 56,2 ; H 3,7 ; N 3,1 ; Cl 24,8
UV (in DMF): 399 nm (22700)

H O E C H S T   A K T I E N G E S E L L S C H A F T
KALLE Niederlassung der Hoechst AG



Verbindung 21:

2-Trichloracetylmethylen-3-ethyl-4,5-diphenyl-thiazolin
Schmp.: 161-162°C

$C_{20}H_{16}Cl_3NOS$ Ber.: C 56,55; H 3,80; N 3,30; Cl 25,04
MG: 424,78 Gef.: C 56,8 ; H 3,8 ; N 3,2 ; Cl 24,8
UV (in DMF): 292 nm (S, 7200), 380 nm (25800)

Verbindung 21a: 2-Trichloracetylmethylen-3-ethyl-5-phenyl-thiazolin
Schmp.: 144-145°C

$C_{14}H_{12}Cl_3NOS$: Ber: C 48,23; H 3,47; N 4,02; Cl 30,50
MG: 348,68 Gef: C 48,1 ; H 3,7 ; N 4,0 ; Cl 30,2
UV (in DMF): 366 nm (24400)

Herstellungsbeispiel 6

Herstellung der Verbindungen 22 und 23:

A) 1,2-Dimethyl-chinolinium-p-toluolsulfonat:

71,5 g (0,5 mol) Chinaldin und 102,3 g (0,55 mol) p-Toluolsulfonsäuremethylester werden in ca. 10 Minuten auf 100°C erwärmt. Die einsetzende exotherme Reaktion bringt das Reaktionsgemisch auf 180°C. Nach 10 Minuten wird auf Aceton gegossen, abgesaugt, mit Aceton gewaschen und getrocknet.
Ausbeute: 151 g (0,46 mol = 92 %)

HOECHST AKTIENGESELLSCHAFT
KALLE Niederlassung der Hoechst AG



B) 2-Trichloracetylmethylen-1-methyl-1,2-dihydrochinolin und 2-(Bis-trichloracetyl-methylen)-1-methyl-1,2-dihydrochinolin

Zu 10 g (30 mmol) 1,2-Dimethyl-chinolinium-p-toluolsulfonat in 25 ml Pyridin werden bei 0-5°C 6 g (33 mmol) Trichloracetylchlorid getropft. Nach 2 Stunden bei Raumtemperatur wird mit Methylenchlorid versetzt, die Lösung mit Wasser gewaschen, über Natriumsulfat getrocknet, konzentriert und zweimal mit Cyclohexan/Ethylacetat (1:1) an Kieselsäuregel chromatographiert.

1. eluierte Zone: 300 mg
Verbindung 22: 2-Trichloracetyl-1-methyl-1,2-dihydrochinolin
Schmp.: 228-230°C

$C_{13}H_{10}Cl_3NO$ Ber.: C 51,60; H 3,33; N 4,63; Cl 35,15
MG: 302,59 Gef.: C 51,6 ; H 3,3 ; N 4,4 ; Cl 35,1
UV (in DMF): 305 nm (12500), 402 nm (S, 21200)
419 nm (30200), 441 nm (23700)

2. eluierte Zone: 200 mg
Verbindung 23: 2-(Bis-trichloracetyl-methylen)-1-methyl-1,2-dihydrochinolin
Schmp.: 175-176°C

$C_{15}H_9Cl_6NO_2$ Ber.: C 40,22; H 2,03; N 3,13; Cl 47,49
MG: 447,96 Gef.: C 40,1 ; H 2,0 ; N 2,7 ; Cl 46,8
UV (in DMF): 323 nm (13900), 442 nm (7100)

H O E C H S T   A K T I E N G E S E L L S C H A F T
KALLE Niederlassung der Hoechst AG



Anwendungsbeispiel 1:

Auf eine mechanisch aufgerauhte Aluminiumplatte wird eine Lösung bestehend aus

| | | |
|---|---|---|
| 0,5 | Gt | Verbindung 1, |
| 23,75 | " | eines Polyacetals aus Triethylenglykol und 2-Ethyl-butyraldehyd und |
| 75,0 | Gt | Kresol-Formaldehyd-Novolak (Schmelzbereich 105-120°C nach Kapillarmethode DIN 53 181) in |
| 24,25 | " | 2-Ethoxy-ethanol und |
| 375 | Vt | Methylethylketon |

aufgeschleudert und bei 100 Grad getrocknet. Es wird durch einen Stufenkeil belichtet, bei dem sich die optische Dichte einer Stufe um den Faktor $\sqrt{2}$ von der der nächstfolgenden Stufe unterscheidet, und das positive Bild mit einer Lösung aus

| | | |
|---|---|---|
| 5,5 | Gt | Natriummetasilikat · 9 $H_2O$, |
| 3,4 | " | Trinatriumphosphat · 12 $H_2O$, |
| 0,4 | " | Natriumdihydrogenphosphat (wasserfrei) und |
| 90,7 | " | entsalztem Wasser |

entwickelt. Tabelle VII zeigt, daß die Anzahl der entwickelten Keilstufen jeweils bei Verdoppelung der Belichtungszeit um 2 zunimmt.

Tabelle VII

| Belichtungszeit (min) | entwickelte Keilstufen |
|---|---|
| 0,5 | 3 |
| 1 | 5 |
| 2 | 7 |
| 4 | 9 |

Anwendungsbeispiel 2:

Dieses Beispiel zeigt, daß unter den erfindungsgemäßen Verbindungen diejenigen mit einer p Trichlormethylbenzoyl-Gruppe besonders empfindlich sind. Die aus der USA Defensive Publication T 900011-Q bekannten Trifluormethylbenzoylmethylen-thiazole (Verbindungen 5 und 11) und die aus den DE-B 27 17 778 bekannten Verbindungen ohne Trihalogenmethylgruppe sind dagegen wirkungslos.

Die Durchführung erfolgt entsprechend Anwendungsbeispiel 1, wobei in dem Beschichtungsgemisch Verbindung 1 durch die gleiche Menge einer der in Tabelle VIII angegebenen Verbindungen ersetzt wird.

Tabelle VIII

| Verbindung Nr. | entwickelte Keilstufen bei einer Belichtungszeit von | |
|---|---|---|
| | 2 Minuten | 4 Minuten |
| 1 | 7 | 9 |
| 1a | 5 | 7 |
| 1b | 5 | 8 |
| 2 | 1 | 3 |
| 3 | 0 | 1 |
| 4 | 7 | 9 |
| 6 | 5 | 8 |
| 6a | 5 | 7 |
| 6b | 5 | 7 |
| 6c | 5 | 8 |
| 7 | 0 | 1 |
| 8 | 6 | 8 |
| 8a | 5 | 8 |
| 10 | 6 | 8 |
| 10a | 0 | 2 |
| 13 | 5 | 7 |
| 15 | 5 | 7 |
| 15a | 4 | 5 |
| 15b | 0 | 0 |
| 16 | 2 | 4 |
| 16a | 3 | 6 |
| 17 | 0 | 1 |
| 18 | 4 | 6 |
| 19 | 5 | 7 |

H O E C H S T   A K T I E N G E S E L L S C H A F T
KALLE Niederlassung der Hoechst AG



Tabelle VIII (Fortsetzung)

| Verbindung Nr. | entwickelte Keilstufen bei einer Belichtungszeit von | |
|---|---|---|
| | 2 Minuten | 4 Minuten |
| 20 | 2 | 4 |
| 20a | 3 | 5 |
| 21 | 2 | 3 |
| 21a | 3 | 5 |
| 22 | 0 | 1 |
| 23 | 0 | 0 |
| 24 | 5 | 8 |
| 25 | 6 | 8 |
| 26 | 5 | 8 |
| 27 | 5 | 8 |

| Vergleichsverbindungen mit $CF_3$-Gruppe | Belichtungszeit 8 Minuten | Belichtungszeit 16 Minuten |
|---|---|---|
| 5 | 0 | 0 |
| 11 | 0 | 0 |

| ohne $CX_3$-Gruppe | Belichtungszeit 8 Minuten | Belichtungszeit 16 Minuten |
|---|---|---|
| 9 | 0 | 0 |
| 12 | 0 | 0 |
| 14 | 0 | 0 |

| mit $CCl_3$-Gruppe | Belichtungszeit 2 Minuten | Belichtungszeit 4 Minuten |
|---|---|---|
| A | 6 | 9 |
| B | 7 | 9 |
| C | 6 | 8 |

A = 2-(p-Methoxyphenyl)-4-chlor-5-(p-trichlormethylphenyl)-oxazol

B = 2-(p-Trichlormethylphenyl)-4-chlor-5-(p-methoxyphenyl)-oxazol

C = 2,4-Bis(trichlormethyl)-6-(4-ethoxynaphth-1-yl)-s-triazin

HOECHST AKTIENGESELLSCHAFT
KALLE Niederlassung der Hoechst AG



Anwendungsbeispiel 3

Eine einseitig durch Drahtbürsten mechanisch aufgerauhte Aluminiumplatte wurde mit folgender Lösung beschichtet:

| | | |
|---|---|---|
| 3 | Gt | des im Anwendungsbeispiel 1 angegebenen Novolaks, |
| 1 | " | eines polymeren Acetals aus 2-Ethylbutyraldehyd und Hexan-1,6-diol, |
| 0,001 | " | Kristallviolettbase und |
| 0,02 | " | Verbindung 10 in |
| 96 | " | eines Lösungsmittelgemisches aus Ethylenglykolmonoethylether und Butylacetat (4 : 1). |

Die getrocknete Schicht hatte ein Schichtgewicht von 2 $g/m^2$. Sie wurde unter einer positiven Vorlage 100 Sekunden mit einer 5 kW-Metallhalogenidlampe im Abstand 140 cm belichtet, wobei ein Bildkontrast durch Aufhellung der belichteten Partien entstand. Diese wurden mit einer Lösung aus

| | | |
|---|---|---|
| 2,67 | Gt | Natriummetasilikat · 9 Wasser, |
| 1,71 | " | Trinatriumphosphat · 12 Wasser und |
| 0,17 | " | Mononatriumphosphat (wasserfrei) in |
| 95,45 | " | vollentsalztem Wasser |

ausgewaschen. Danach wurde wie üblich mit Wasser abgespült und durch Überwischen mit 1 %iger Phosphorsäure druckfertig gemacht. Es wurde eine leistungsfähige Flachdruckplatte erhalten.

H O E C H S T   A K T I E N G E S E L L S C H A F T
KALLE Niederlassung der Hoechst AG



Anwendungsbeispiel 4

Eine Lösung wurde hergestellt aus

| | | |
|---|---|---|
| 6,5 | Gt | eines Terpolymerisats aus n-Hexylmethacrylat, Methacrylsäure und Styrol (60:30:10) mit einem mittleren Molgewicht von ca. 35.000 und der Säurezahl 195, |
| 3,2 | " | Polyethylenglykol-400-dimethacrylat, |
| 0,1 | " | der Verbindung 15 und |
| 0,04 | " | eines Azofarbstoffs, hergestellt durch Kuppeln von 2,4-Dinitro-6-chlor-benzoldiazoniumsalz mit 2-Methoxy-5-acetylamino-N-cyanoethyl-N-hydroxyethyl-anilin in |
| 25 | " | Methylethylketon und |
| 3 | " | Ethanol. |

Diese Beschichtungslösung wurde auf eine biaxial verstreckte und thermofixierte Polyethylenterephthalatfolie der Stärke 25 µm so aufgeschleudert, daß nach dem Trocknen bei 100°C ein Schichtgewicht von 45 $g/m^2$ erhalten wurde. Die so erhaltene Trockenresistfolie wurde mit einem handelsüblichen Laminator bei 120°C auf eine mit 35 µm dicker Kupferfolie kaschierte Phenoplast-Schichtstoffplatte laminiert und 40 Sekunden unter einer Leiterplattenvorlage belichtet. Nach der Belichtung wurde die Polyesterfolie abgezogen, und die unbelichteten Schichtteile wurden in einem Sprühgerät mit 1 %iger Sodalösung innerhalb 1 Minute ausgewaschen.

Nach Spülen mit Wasser und Anätzen mit 15 %iger Ammoniumperoxydisulfat-Lösung wurde an den freigelegten Stellen nacheinander in dem Glanzkupferbad und dem Nickelbad "Norma" der Fa. Schlötter und dem Goldbad "Autronex XX" der Fa. Blasberg jeweils mit den vom Hersteller empfohlenen Stromdichten und Schichtdicken galvanisiert. Nach Entfernen der Resistschablone mit 5 %iger KOH-Lösung bei 40-50°C und Wegätzen des so freigelegten Basiskupfers mit üblichen Ätzmitteln wurde eine gute Leiterplatte erhalten.

Anwendungsbeispiel 5

Zur Herstellung eines Positiv-Trockenresists wurde die folgende Lösung hergestellt:

| | | |
|---|---|---|
| 64,75 | Gt | Methylethylketon, |
| 21,2 | " | des im Anwendungsbeispiel 1 beschriebenen Novolaks, |
| 10 | " | des Bis-(5-ethyl-5-methoxymethyl-1,3-dioxolan-2-yl)ethers von 2-Ethyl-2-methoxymethyl-1,3-propandiol, |
| 3,8 | " | Polyethylacrylat, niedrig-viskos, |
| 0,05 | " | Kristallviolettbase und |
| 0,2 | " | der Verbindung 4. |

Damit wurde eine mit Trichloressigsäure/Polyvinylalkohol-Lösung vorbehandelte biaxial verstreckte und thermofixierte 25 µm dicke Polyesterfolie analog Anwendungsbeispiel 4 beschichtet, getrocknet und beidseitig auf Kupferblech laminiert. Nach Abkühlen, Abziehen der Trägerfolie und kurzem Nachtrocknen im Trockenschrank

bei 80°C wurde das beschichtete Blech mit einem deckungsgleichen Vorlagenpaar in Form einer Tasche beidseitig belichtet. Wie im Anwendungsbeispiel 1 wurden dann die belichteten Schichtpartien entfernt, in diesem Fall durch beidseitiges Besprühen mit dem Entwickler. Bei einem 25 µm dicken Resist wurde 40 Sekunden belichtet und 45 Sekunden entwickelt, bei 12 µm Resistdicke genügten 25 Sekunden Belichtung und 25 Sekunden Entwicklung. Bei beiden Resistdicken wurden die Platten nach Abspülen der alkalischen Entwicklerreste mit handelsüblicher Ferrichlorid-Lösung beidseitig so lange geätzt, bis sie sauber durchgeätzt waren. Die erhaltenen Formätzteile können noch mit ca. 3 %iger KOH- oder NaOH-Lösung oder Aceton von der Resistschablone befreit werden.

Anwendungsbeispiel 6

Für die Herstellung eines Negativ-Trockenresists wird auf eine Trägerfolie wie im Anwendungsbeispiel 4 eine Lösung folgender Zusammensetzung aufgebracht:

| | | |
|---|---|---|
| 68,5 | Gt | Methylethylketon, |
| 19 | " | eines Copolymeren aus Polymethylmethacrylat/ Methacrylsäure (98:2) mit einem Molekulargewicht von ca. 34.000, |
| 12 | " | Trimethylolpropantriacrylat, |
| 0,2 | " | Verbindung 1, |
| 0,2 | " | Leuko-Kristallviolett, |
| 0,1 | " | Malachitgrün. |

HOECHST AKTIENGESELLSCHAFT
KALLE Niederlassung der Hoechst AG



Die erhaltene gleichmäßige photopolymerisierbare Schicht der Dicke 38 µm wurde auf eine mit Kupferfolie kaschierte Schichtstoffplatte laminiert und dann 20 Sekunden in der im Anwendungsbeispiel 3 angegebenen Belichtungseinrichtung belichtet. Dabei wird durch die lichtempfindliche Trichlormethylphenyl-Verbindung sowohl die Photopolymerisation initiiert als auch die Farbbildung von Kristallviolett aus der Leuko-Verbindung hervorgerufen.

Wird anstelle der Verbindung 1 die entsprechende Trifluormethyl-Verbindung 5 in gleicher Weise eingesetzt, so tritt überhaupt keine Reaktion an den belichteten Stellen ein, auch nicht bei verlängerter Belichtungszeit. Wenn man dagegen die Verbindung 1 durch die Verbindung 2 ersetzt, so erhält man eine deutlich geringere Lichtempfindlichkeit. Während mit Verbindung 1 der Halbtonstufenkeil bis Stufe 8 gedeckt ist, zeigt die entsprechende Schicht mit Verbindung 2 bei gleicher Belichtung nach 1 Minute Sprühentwicklung mit Trichlorethan nur bis zu Stufe 2 ausreichende Deckung. Wird die Verbindung 1 durch die Vergleichsverbindung A (Anwendungsbeispiel 2) ersetzt, so erhält man kein Bild.

Ein ähnliches Ergebnis wie mit Verbindung 1 als Starter wird erhalten, wenn als Vergleich die Verbindung 2-(4-Ethoxy-naphth-1-yl)-4,6-bis-trichlormethyl-s-triazin verwendet wird. Die Lagerfähigkeit dieses Resists auf Kupfer laminiert, ist jedoch deutlich schlechter. Das ergibt sich aus der beschleunigten Lagerfähigkeitsprüfung bei 40°C. Während das Material mit dem bekannten Trichlormethyltriazin als Initiator nach 8 Tagen Lagerung und anschließender Belichtung und Entwicklung einen um 3 Keilstufen kürzeren Halbtonstufenkeil ergibt, wird unter sonst gleichen Bedingungen bei Verwendung der Verbindung 1 der Keil nur um etwa 1 Stufe verkürzt. Nach der gleichen Lagerung zeigt das unbelichtete Material mit dem bekannten Initiator eine deutliche Blaufärbung, während diese bei dem erfindungsgemäßen Material nur gering ist.

Anwendungsbeispiel 7

Eine Lösung von

| | | |
|---|---|---|
| 3 | Gt | des im Anwendungsbeispiel 1 angegebenen Novolaks, |
| 1 | " | eines polymeren Acetals aus 2-Ethylbutyraldehyd und Triethylenglykol, |
| 0,002 | " | Kristallviolettbase und |
| 0,6 | " | der Verbindung 1 in |
| 76 | " | 2-Ethoxy-ethan und |
| 19,4 | " | Butylacetat |

wurde auf einen Schichtträger aus elektrolytisch aufgerauhtem und anodisch oxydiertem Aluminium aufgeschleudert und getrocknet. Das Schichtgewicht betrug 2,5 g/m$^2$. Die erhaltene Druckplatte wurde wie im Anwendungsbeispiel 3 belichtet und mit der im Anwendungsbeispiel 1 angegebenen Lösung entwickelt.

H O E C H S T   A K T I E N G E S E L L S C H A F T
KALLE Niederlassung der Hoechst AG



Anwendungsbeispiel 8

Eine Lösung von

| | | |
|---|---|---|
| 19 | Gt | eines Terpolymerisats aus Butylmethacrylat, Methylmethacrylat und Methacrylsäure (78:20:2, Molgewicht ca. 100 000), |
| 12 | " | Dipentaerythrithexaacrylat, |
| 0,2 | " | Verbindung 13 und |
| 0,2 | " | Leukokristallviolett in |
| 68,6 | " | Butanon |

wurde auf eine mit 35 µm dicker Kupferfolie kaschierte Phenoplast-Schichtstoffplatte so aufgeschleudert, daß nach dem Trocknen bei 100°C eine Schicht von 25 µm Dicke erhalten wurde. Die Platte wurde in der im Anwendungsbeispiel 3 angegebenen Belichtungseinrichtung 30 Sekunden belichtet und durch Sprühen mit Trichlorethan entwickelt. Das Schaltbild wurde in üblicher Weise durch Galvanisieren, Entschichten und Ätzen des Basiskupfers weiterverarbeitet.

Anwendungsbeispiel 9

Eine Lösung aus

| | | |
|---|---|---|
| 6,5 | Gt | des im Anwendungsbeispiel 4 angegebenen Terpolymerisats, |
| 2,8 | " | eines polymerisierbaren Diurethans, das durch Umsetzung von 1 mol 2,2,4-Trimethylhexamethylendiisocyanat mit 2 mol Hydroxyethylmethacrylat erhalten wurde, |

HOECHST AKTIENGESELLSCHAFT
KALLE Niederlassung der Hoechst AG



| | | |
|---|---|---|
| 2,8 | Gt | eines polymerisierbaren Polyurethans, das durch Umsetzung von 11 mol 2,2,4-Trimethylhexamethylendiisocyanat mit 10 mol wasserfreiem Triethylenglykol und weitere Umsetzung des Reaktionsprodukts mit 2 mol Hydroxyethyl-methacrylat hergestellt wurde, |
| 0,2 | " | der Verbindung 4, |
| 0,1 | " | 3-Mercapto-propionsäure-2,4-dichlor-anilid, |
| 0,035 | " | des im Anwendungsbeispiel 4 angegebenen blauen Azofarbstoffs und |
| 2,8 | " | des Esters von 2,6-Dihydroxy-benzoesäure mit Diethylenglykol-mono-2-ethylhexylether |
| in 35 | " | Methylethylketon und |
| 2 | " | Ethanol |

wurde auf eine biaxial verstreckte und thermofixierte Polyethylenterephthalatfolie der Stärke 25 µm so aufgeschleudert, daß nach dem Trocknen bei 100°C ein Schichtgewicht von 28 $g/m^2$ erhalten wurde.

Die erhaltene Trockenresistfolie wurde mit einer handelsüblichen Laminiervorrichtung bei 120°C auf eine mit 35 µm starker Kupferfolie kaschierte Phenoplast-Schichtstoffplatte laminiert und 20 Sekunden mit einem handelsüblichen Belichtungsgerät belichtet. Als Vorlage diente eine Strichvorlage mit Linienbreiten und Abständen bis herab zu 80 µm.

Nach der Belichtung wurde die Polyesterfolie abgezogen und die Schicht in einer 0,8 %igen $Na_2CO_3$-Lösung in einem Sprühentwicklungsgerät 50 Sekunden lang entwickelt.

H O E C H S T   A K T I E N G E S E L L S C H A F T
KALLE Niederlassung der Hoechst AG



Die Platte wurde dann 30 Sekunden mit Leitungswasser gespült, 1 Minute in einer 25 %igen Ammoniumperoxydisulfat-Lösung angeätzt und sodann nacheinander in den folgenden Elektrolytbädern galvanisiert:

1.) 40 Minuten in einem Kupferelektrolytbad der Firma Blasberg, Solingen, Typ "Feinkornkupferplastic-Bad"
Stromdichte: 2 A/$dm^2$
Metallaufbau: ca. 20 µm

2.) 10 Minuten in dem im Anwendungsbeispiel 4 angegebenen Nickelbad
Stromdichte: 4 A/$dm^2$
Metallaufbau: 6 µm und

3.) 15 Minuten in einem Goldbad Typ "Autronex N" der Firma Blasberg, Solingen
Stromdichte: 0,6 A/$dm^2$
Metallaufbau: 2,5 µm

Die Platte zeigt keinerlei Unterwanderungen oder Beschädigungen.

Die Platte kann sodann in 5 %iger KOH-Lösung bei 50°C entschichtet und das freigelegte Kupfer in den üblichen Ätzmedien weggeätzt werden.

HOECHST AKTIENGESELLSCHAFT
KALLE Niederlassung der Hoechst AG



## Patentansprüche

1. Verbindungen der allgemeinen Formel I

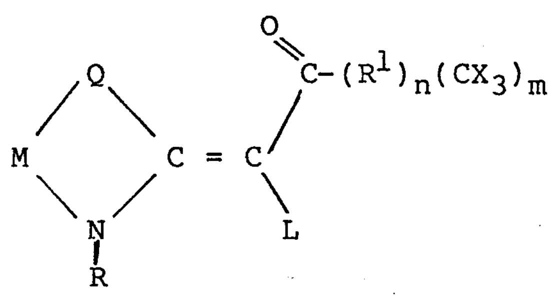

worin

L ein Wasserstoffatom oder einen Substituenten der Formel $CO-(R^1)_n(CX_3)_m$,

M einen substituierten oder unsubstituierten Alkylenrest oder Alkenylenrest oder einen 1,2-Arylenrest,

Q ein Schwefel-, Selen- oder Sauerstoffatom, eine Dialkylmethylengruppe, einen Alken-1,2-ylenrest, einen 1,2-Phenylenrest oder eine Gruppe N-R,

wobei M + Q zusammen 3 oder 4 Ringglieder bilden,

R einen Alkyl-, Aralkyl- oder Alkoxyalkylrest,

$R^1$ eine carbo- oder heterocyclische aromatische Gruppe,

X ein Chlor-, Brom- oder Jodatom bedeutet und

n = O und m = 1 oder

n = 1 und m = 1 oder 2 ist.

H O E C H S T   A K T I E N G E S E L L S C H A F T
KALLE Niederlassung der Hoechst AG



2. Verbindungen nach Anspruch 1, dadurch gekennzeichnet, daß X ein Chloratom ist.

3. Verbindungen nach Anspruch 1, dadurch gekennzeichnet, daß $R^1$ ein Phenylen- oder Phentriylrest ist.

4. Verbindungen nach Anspruch 1, dadurch gekennzeichnet, daß Q ein Schwefelatom ist.

5. Lichtempfindliches Gemisch, enthaltend eine lichtempfindliche heterocyclische organische Verbindung (a) mit mindestens einem Trihalogenmethylsubstituenten und eine Verbindung (b), die mit dem Lichtreaktionsprodukt der organischen Verbindung (a) unter Ausbildung eines Produktes zu reagieren vermag, das eine von (b) unterschiedliche Lichtabsorption oder Löslichkeit in einem Entwickler aufweist, dadurch gekennzeichnet, daß die organische Verbindung (a) eine Verbindung gemäß Anspruch 1 ist.

6. Lichtempfindliches Gemisch nach Anspruch 5, dadurch gekennzeichnet, daß die Verbindung (b) eine ethylenisch ungesättigte Verbindung ist, die eine durch freie Radikale ausgelöste Polymerisation einzugehen vermag.

7. Lichtempfindliches Gemisch nach Anspruch 5, dadurch gekennzeichnet, daß die Verbindung (b) mindestens eine durch Säure spaltbare C-O-C-Bindung enthält.

8. Lichtempfindliches Gemisch nach Anspruch 5, dadurch gekennzeichnet, daß die Verbindung (b) durch Säure zur kationischen Polymerisation angeregt zu werden vermag.

9. Lichtempfindliches Gemisch nach Anspruch 5, dadurch gekennzeichnet, daß die Verbindung (b) durch Säure vernetzbar ist.

10. Lichtempfindliches Gemisch nach Anspruch 5, dadurch gekennzeichnet, daß die Verbindung (b) durch Einwirkung von Säure ihren Farbton ändert.

11. Lichtempfindliches Gemisch nach Anspruch 5, dadurch gekennzeichnet, daß es die Verbindung der Formel I in einer Menge von 0,1 bis 15 Gew.-%, bezogen auf seine nichtflüchtigen Bestandteile, enthält.

12. Lichtempfindliches Gemisch nach Anspruch 5, dadurch gekennzeichnet, daß es zusätzlich ein wasserunlösliches polymeres Bindemittel enthält.

13. Lichtempfindliches Gemisch nach Anspruch 12, dadurch gekennzeichnet, daß das Bindemittel in wäßrig-alkalischen Lösungen löslich ist.

14. Verfahren zur Herstellung einer Verbindung der Formel I gemäß Anspruch 1, dadurch gekennzeichnet, daß man eine Verbindung der Formel II

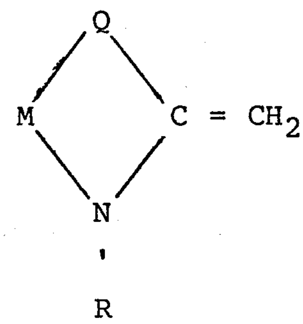

(II)

oder deren Iminiumsalz der Formel III

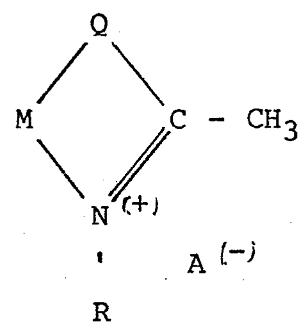

(III)

worin $A^{(-)}$ das Anion des Iminiumsalzes bedeutet, mit einem Carbonsäurehalogenid der Formel IV

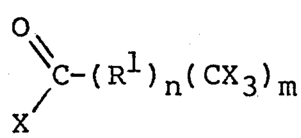

(IV)

umsetzt, wobei die Symbole L, M, Q, R, $R^1$, X, n und m die im Anspruch 1 angegebene Bedeutung haben.

15. Verfahren nach Anspruch 14 zur Herstellung einer Verbindung der Formel I mit L = H, dadurch gekennzeichnet,

HOECHST AKTIENGESELLSCHAFT
KALLE Niederlassung der Hoechst AG



daß man die Verbindung der Formel II oder III mit ungefähr 1 Äquivalent der Verbindung der Formel IV umsetzt.

16. Verfahren nach Anspruch 14 zur Herstellung einer Verbindung der Formel I mit L = $CO(R^1)_n(CX_3)_m$, dadurch gekennzeichnet, daß man die Verbindung der Formel II oder III mit ungefähr 2 Äquivalenten der Verbindung der Formel IV umsetzt.